(19) Europäisches Patentamt
European Patent Office
Office européen des brevets

(11) Publication number: 0 339 669 A1

(12) # EUROPEAN PATENT APPLICATION

(21) Application number: 89107764.6

(22) Date of filing: 28.04.89

(51) Int. Cl.4: C07D 455/00 , C07D 471/18 , //A61K31/445,(C07D471/18, 221:00,221:00,221:00)

(30) Priority: 29.04.88 US 188232

(43) Date of publication of application: 02.11.89 Bulletin 89/44

(84) Designated Contracting States: AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Anzeveno, Peter B.
10040 Barth Drive
Zionsville Indiana 46077(US)

(74) Representative: Vossius & Partner
Siebertstrasse 4 P.O. Box 86 07 67
D-8000 München 86(DE)

(54) **Process for preparing indole-3-carboxylic acid esters of trans-hexahydro-8-hydroxy-2,6-methano-2H-quinolizin-3(4H)-one.**

(57) The present invention describes a process for preparing indole-3-carboxylic acid esters of hexahydro-8-hydroxy-2,6-methano-2H-quinolizin-3(4H)-one which comprises reacting 5-hydroxy-8-azatricyclo[5.3.1.0$^{3,8}$]-undecan-10-one with the mixed anhydride of trifluoroacetic acid and an appropriate indole-3-carboxylic acid.

EP 0 339 669 A1

# PROCESS FOR PREPARING INDOLE-3-CARBOXYLIC ACID ESTERS OF TRANS-HEXAHYDRO-8-HYDROXY-2,6-METHANO-2H-QUINOLIZIN-3(4H)-ONE

Indole-3-carboxylic acid esters of trans-hexahydro-8-hydroxy-2,6-methano-2H-quinolizin-3(4H)-one have been found to be useful for the treatment of migraine and a number of other disorders. However, because of the hindered nature of the alcohol and instability of indoles, many of the ordinary procedures for producing esters do not give any of the desired product and those that do may not be completely satisfactory for a number of reasons. Thus, currently, a preferred procedure for obtaining such compounds involves the reaction of an indolylglyoxylyl chloride with silver tetrafluoroborate and the tetrafluoroborate salt of endohexahydro-8-hydroxy-2,6-methano-2H-quinolizin-3(4H)-one. Use of the expensive silver compound is an obvious disadvantage to this procedure.

Thus, the present invention is directed to a new and convenient preparation of such esters. More particularly, the present invention is directed to a process for preparing esters of the formula

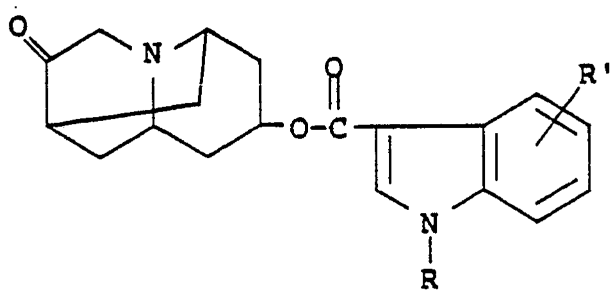

wherein R is hydrogen or $C_{1-4}$ alkyl and R′ is hydrogen halogen or $C_{1-4}$ alkyl, and the pharmaceutically acceptable acid addition salts thereof, which comprises reacting an alcohol of the formula

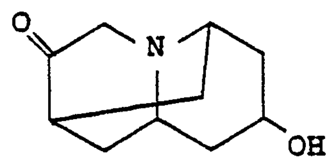

with an anhydride of the formula

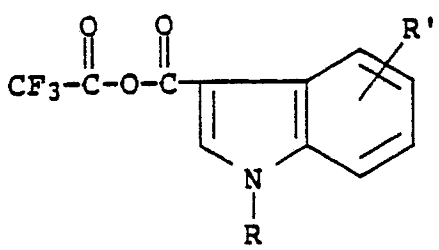

wherein R and R′ are defined as above. The reaction is carried out in an inert solvent at ambient temperature in the presence of a tertiary amine. The procedure conveniently gives pure product in good yields without the use of expensive reagents.

Examples of the $C_{1-4}$ alkyl groups referred to above are methyl, ethyl, propyl and butyl. Hexahydro-8-hydroxy-2,6-methano-2H-quinolizin-3(4H)-one and 5-hydroxy-8-azatricyclo[5.3.1.$0^{3,8}$]undecan-10-one are two alternative names for the tricyclic alcohol shown above. The configuration of the hydroxy group in the alcohol can be either endo or exo although the present process is limited to those in which it is endo. Such endo-compounds can also be referred to as trans-compounds

The inert solvent used should be one in which the reactants and the product are soluble. Methylene chloride has thus been found to be a preferred solvent for the reaction. 4-Dimethylaminopyridine is a preferred tertiary amine.

By ambient temperature is meant room temperature or slightly below room temperature. Specifically, the reaction can be carried out with some cooling to maintain the mixture at room temperature.

The acid anhydride shown above is the mixed anhydride of trifluoroacetic acid with an appropriate indole-3-carboxylic acid. The mixed anhydride can be conveniently prepared *in situ* using trifluoroacetic anhydride and the appropriate indole-3-carboxylic acid.

The compounds prepared herein are useful for the treatment of pain, especially migraine, vascular and cluster headaches and trigeminal neuralgia. They are also useful in the treatment of nausea and vomiting arising from treatment with cancer chemotherapeutic agents.

In the past, acute attacks of migraine have been treated with a peripheral vasoconstrictor, such as ergotamine, which may be co-administered with caffeine, and dihydroergotamine; an antipyretic analgesic, such as acetylsalicylic acid or p-acetylaminophenol; and/or an antiemetic such as cyclizine, metoclopramide and thiethylperazine. It has also been reported (J. B. Hughes, Med. J. Aust. 2, No. 17, 580 (1977)) that immediate relief of an acute migraine attack can be obtained by slow intravenous injection of metoclopramide (10 mg).

It is believed that 5-hydroxytryptamine (5-HT) is the naturally occurring substance most likely to play a role in the pathophysiology of migraine. Increased amounts of 5-HT and it metabolite 5-hydroxyindoleacetic acid are excreted in the urine during most attacks. Further, plasma and platelet 5-HT concentrations fall rapidly at the onset of an attack and remain low while the headache persists. Moreover, attacks of migraine have been clearly associated with periods of thrombocytopaenia in certain patients. It has been proposed that compounds which block the activity of 5H-T would be of use in the symptomatic treatment of migraine (J. R. Fozard, International Headache Congress 1980, reported in Advances in Neurology, Vol 33., Raven Press, New York, 1982).

The known migraine prophylactic drugs, methysergide, propranolol, amitriptyline, and chlcrpromazine have widely different pharmacological activities but all are 5-HT D-receptor antagonists at the doses used clinically for the prophylaxis of migraine. Metoclopramide is a potent 5-HT M-receptor antagonist and it has been proposed (J. R. Fozard supra) that a blockade of the M-receptor present on afferent sensory neurones affords symptomatic relief in an acute migraine attack.

The potency as 5-HT receptor antagonists of (-) cocaine and some related compounds, including pseudotropyl benzoate (i.e., benzoylpseudotropine) and 3,5-dichlorobenzoyltropine has been reported (J. R. Fozard et al., Eur. J. Pharmacol., 59, (1979) 195-210; J. R. Fozard, Naunyn-Schmiedeberg's Arch Pharmacol., 326, (1984), 36-44). The $pA_2$ values reported for metoclopramide, pseudotropyl benzoate, nor (-) cocaine and benzoyltropine are 7.2, 7.0, 7.7, and 7.2 respectively whilst the $pA_2$ value determined for 3,5-dichlorobenzoyltropine by the same procedure is 9.3 (J. R. Fozard et al., Eur. J. Pharmacol., 49, (1978) 109-112; J. R. Fozard, Naunyn-Schmiedeberg's Arch Pharmacol., 326, (1984), 36-44). In a double-blind clinical trial, 3,5-dichlorobenzoyltropine proved an effective treatment for the acute migraine attack (C. Loisy et al., Cephalalgia, 5, (1985) 79-82). A further series of tropine esters, with $pA_2$ values for blockade of the 5-HT M-receptors between 7.7 and 13.6 have been described by Richardson et al., Nature, 316, (1985) 26-131.

The compounds prepared by the present invention block the M-receptors for 5-hydroxytryptamine (5-HT) on afferent sensory neurones, certain of which subserve the transmission of pain. As explained above, the blocking of such M-receptors appears to be a mechanism whereby the symptoms of migraine can be relieved. Accordingly, the present copounds are useful in the treatment of migraine when administered in amounts sufficient to effectively block the said M-receptors.

In addition, compounds blocking 5-HT M-receptors, including metoclopramide, 3,5-dichlorobenzoyltropine and (3α-tropanyl)-1H-indole-3-carboxylic acid ester, are highly effective in preventing the nausea and vomiting induced by cancer chemotherapeutic agents in an animal experimental model (W. D. Miner et al., Brit. J. Pharmacol., 88, (1986) 374P; W. D. Miner and G. J. Sanger, Brit J. Pharmacol., 88, (1986) 497-499; B. Costall et al., Neuropharmacology, 25, (1986) 959-961). It is believed that cytotoxic drug-induced vomiting involves a 5-HT M-receptor mechanism (W. D. Miner and G. J. Sanger, Brit J. Pharmacol., 88, (1986) 497-499). Accordingly, the present compounds are useful in the treatment of cytotoxic drug-induced vomiting when administered in amounts sufficient to effectively block the said M-receptors.

The activity of the compounds prepared herein against 5-HT can be assessed by determining their $pA_2$ values in the isolated rabbit heart as described by J.R. Fozard et al., Eur. J. Pharmacol., 59, 195-210 (1979). In the method described, the molar concentration of antagonist which reduces the effects of twice the $ED_{50}$ of 5-HT to that of the $ED_{50}$ in the absence of antagonist is determined. The $pA_2$ value is the negative logarithm of said molar concentrtions. In general terms, the higher the $pA_2$ value the more potent is the compound. When tested in this way, the present compounds show $pA_2$'s generally in the range of about 8 to 10.

The activity of these compounds against 5-HT can be asessed in vivo by measurement of the effect of

the compound on the Von Bezold-Jarisch Reflex induced by 5-HT injected intravenously into the rat (see Paintal A.S., Physiol. Rev., 53, 159-227 (1973); J.R. Fozard, Naunyn-Schmiedeberg's Arch. Pharmacol., 326, (1984) 36-44). The transient cardiac slowing arises from an increased afferent vagus activity arising from stimulation by 5-HT of sensory afferent fibers in and around the heart. When tested against the Von Bezold-Jarisch Reflex induced by 5-HT, the compound endohexahydro-8-(3-indolylcarbonyloxy)-2,6-methano-2H-quinolizin-3(4H)one hydrochloride suppressed the response dose-dependently at doses of 0.01-0.1 mg/kg given intravenously or 0.25-1 mg/kg given orally.

The compounds prepared herein appear to be highly selective in their action against the 5-HT M-receptor. Their potency against other 5-HT receptors and other spasmogens, in particular carbachol, phenylephrine, histamine and calcium, is known to be at least three orders lower that against 5-HT M-receptors. Accordingly, their use in the treatment of migraine or cytotoxic drug-induced vomiting should be without any side effects.

The compounds prepared herein can be administered in various manners to achieve the desired effect. The compounds can be administered alone or in the form of pharmaceutical preparations to the patient being treated either orally or parenterally, for example, subcutaneously or intravenously. They can also be administered by inhalation or by suppository. The amount of compound administered will vary and can be any effective migraine-relieving amount or amount effective in cytotoxic drug vomiting. Depending upon the patient and the mode of administration, the quantity of compound administered may vary over a wide range to provide from about 0.01 mg/kg to about 10 mg/kg, usually 0.03 to 3.0 mg/kg, of body weight of the patient per dose. Unit doses of these compounds can contain, for example, from about 0.5 mg to 100 mg, usually 1 to 50 mg and preferably 3 to 30 mg, of the compound and may be administered, for example, from 1 to 4 times daily.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

Specific formulations of the compounds are prepared in a manner well known per se in the pharmaceutical art and usually comprise one or more active compounds of the invention in admixture or otherwise in association with a pharmaceutically acceptable carrier or diluent therefor. The active ingredient will usually be mixed with a carrier, or diluted by a diluent, or enclosed or encapsulated in a capsule, sachet, cachet, paper or other container. A carrier or diluent may be solid, semisolid or liquid material which serves as a vehicle, excipient or medium for the active ingredient. Suitable carriers or diluents are well known per se. See Remington's Pharmaceutical Sciences, Mack Publishing Company, Easton, Pennsylvania, for a description of the preparation of such formulations.

The formulations of the compounds may be adapted for enteral or parenteral use and may be administered to the patient in the form of tablets, capsules, suppositories, solutions, suspensions or the like.

The esters prepared herein can be used in migraine therapy in combination with other antimigraine drugs having different modes of action. Such drugs include those used prophylactically, such as barbiturates, diazepam, chlorpromazine, amitriptyline, propranolol, methysergide, pizotifen, cyproheptadine, dihydroergotamine, and clonidine, and those used in the acute attack, such as vasoconstrictor agents, e.g., ergotamine and dihydroergotamine, analgesic/ anti-inflammatory agents, e.g., aspirin, paracetamol and indomethacin, or antinauseants, e.g., cyclizine, metoclopramide, and thiethylperazine (see J. F. Fozard, J. Pharm. Pharmaco., 27, 297-321 (1975); J. R. Saper, J. Amer. Med. Assoc., 239, 480-484 (1978); J. R. Fozard, supra). As an example, compounds prepared herein would be beneficial in combination with aspirin 300-1200 mg or methysergide 2-6 mg given daily.

The following examples are presented to illustrate the present invention, but they should not be construed as limiting it in any way.

## EXAMPLE 1

To a stirred, nitrogen blanketed suspension of 4.0 g (0.5 mol) of lithium hydride in 150 ml of dry dimethylformamide, cooled to 0 °C, a solution of 26.4 g (0.2 mol) of dimethyl malonate in 100 ml of dimethylformamide was added dropwise during 30 minutes. After the evolution of hydrogen had essentially ceased (3 hours), 24.0 ml (28.6 g) of cis-1,4-dichloro-2-butene was added during 5 minutes. After 2 hours at

0-5° C, the reaction was stirred at ambient temperature. After 72 hours, the resulting mixture was carefully poured onto 400 ml of saturated sodium chloride solution (caution: foaming and hydrogen evolution) and then extracted with three 300-ml portions of 25% ether in hexane. The organic extracts were washed with saturated sodium chloride (100 ml) and dried over sodium sulfate. Evaporation left 34.3 g of crude dimethyl 3-cyclopentene-1,1-dicarboxylate which was contaminated by 5% of dimethyl 2-vinyl-cyclopropane-1,1-dicarboxylate. This mixture crystallized on standing. Recrystallization from 250 ml of hexane gave 27.8 g (76%) of pure dimethyl 3-cyclopentene-1,1-dicarboxylate as white needles, m.p. 58-59° C.

## EXAMPLE 2

### Method A

A well stirred mixture of 9.2 g (0.05 mol) of dimethyl 3-cyclopentene-1,1-dicarboxylate, 3.6 g (0.06 mol) of sodium chloride, 1.8 ml (0.1 mol) of water and 30 ml of dimethyl sulfoxide was heated, under nitrogen, in an oil bath to 160-165° C and kept at this temperature for 18 hours. The progress of the reaction could be conveniently monitored by gas chromatographic analysis. The dark mixture was then cooled to room temperature, poured onto 200 ml of saturated sodium chloride solution and extracted with ether (3 x 100 ml). The combined extracts were washed with saturated aqueous sodium chloride (50 ml), dried over magnesium sulfate and evaporated, leaving 5.1 g (79%) of crude methyl 3-cyclopentene-1-carboxylate. Distillation gave 4.2 g (70%) of pure material, b.p. 147-148° C, as a colorless oil.

### Method B

A solution of 26.0 g (0.39 mol as 100%) of 85% potassium hydroxide pellets in 280 ml of methanol was added dropwise with stirring, under nitrogen, to a solution of 76.8 g (0.41 mol) of dimethyl 3-cyclopentene-1,1-dicarboxylate in 280 ml of methanol during 1 hour. The resulting mixture was stirred for an additional 2 hours at room temperature after which the methanol was removed at reduced pressure. The solid residue was dissolved in 125 ml of water and this solution was cooled to 0-5° C and acidified by the dropwise addition of 82 ml of 5N hydrochloric acid, added over 30 minutes while maintaining the reaction temperature below 10° C. The mixture was then extracted with ether (3 x 300 ml) and dried over magnesium sulfate. Evaporation of the ether left 64.0 g (92%) of methyl 3-cyclopentene-1-carboxy-1-carboxylate as a sticky, white powder.

The isolated methyl 3-cyclopentene-1-carboxy-1-carboxylate was charged to a 3-necked, 100 ml round-bottomed flask set for downward distillation. Powdered glass (12.0 g) was then added and this mixture heated to 155-160° C in an oil bath under a sweep of dry nitrogen and with good stirring at which point decarboxylation commenced. Clean methyl 3-cyclopentene-1-carboxylate distills as it is produced, b.p. 147-148° C. The pot temperature was slowly raised to 180° C to ensure that all of the produced methyl 3-cyclopentene-1-carboxylate distilled. In this manner, 40.2 g (78% for the 2 steps) of pure methyl 3-cyclopentene-1-carboxylate as a colorless oil, was collected.

## EXAMPLE 3

### Method A

A well stirred solution of 31.5 g (0.24 mol) of methyl 3-cyclopentene-1-carboxylate in 300 ml of methanol:water (2:3) was ozonized at -5° C to 0° C until no methyl 3-cyclopentene-1-carboxylate was detected by gas chromatographic analysis of an aliquot (about 2 hours). Exit gases were passed through 2 liter of a quenching solution prepared from 500 g of sodium thiosulfate, 10 g of potassium iodide and 4 liter of water. The solution was then purged with oxygen for about 10 minutes to remove excess ozone, after which dimethyl sulfide (150 ml) was added. Stirring was continued for 15 minutes, then excess dimethyl sulfide and some methanol were removed at 25-30° C under reduced pressure (about 25 minutes).

The remaining colorless solution of dialdehydo-ester was then diluted with water (100 ml) and, with good stirring, potassium hydrogen phthalate (255.0 g), glycine methyl ester hydrochloride (37.6 g) and acetone-1,3-dicarboxylic acid (43.8 g) were added sequentially. The reaction mixture was stirred under nitrogen for 20 hours during which carbon dioxide was evolved. The mixture was then basified to pH 8-8.5 by the addition of 10% aqueous potassium carbonate solution and extracted with ethyl acetate (3 x 500 ml). Evaporation of the dried (magnesium sulfate) extracts left 40.2 g (60%) of 7-methoxycarbonyl-9-(methoxycarbonylmethyl)-9-azabicyclo[3.3.1]nonane-3-one as an amber colored oil which was greater than 95% pure by thin layer chromatography (15% acetone in methylene chloride) and NMR analysis.

METHOD B

To a mechanically stirred suspension of 72.0 g (0.317 mol) of periodic acid in 450 ml of ethyl acetate cooled to 5-10 °C there was added a solution of 45.0 g (0.317 mol) of 1-methoxycarbonyl-3-cyclopentene oxide in 45 ml of ethyl acetate. The mixture was maintained at about 10 °C until no starting oxide remained as evidenced by gas chromatographic analysis of an aliquot (about 1-1/4 hours). The mixture was then filtered through a pad of Celite into a flask containing 12.5 g of poly(4-vinylpyridine). This mixture was stirred vigorously for 10 minutes at room temperature then filtered through Celite into 1.1 liter of water. The ethyl acetate was evaporated from the water at reduced pressure (about 20 mm) at 30-35 °C. After all of the ethyl acetate was removed, a small amount of water-insoluble oil coated the sides of the distilling flask. The aqueous solution of dialdehydo-ester was decanted from this oil into a 3 liter reaction flask. To this solution was added sequentially, with good stirring, 323.1 g (1.58 mol) of potassium hydrogen phthalate, 47.7 g (0.38 mol) of glycine methyl ester hydrochloride and 55.4 g (0.38 mol) of acetone-1,3-dicarboxylic acid. This mixture was stirred for 18 hours at room temperature, then basified to pH 8.4 with 5N sodium hydroxide, saturated with sodium chloride and extracted with ethyl acetate (5 x 500 ml). The organic extracts were then washed with brine, dried over magnesium sulfate and concentrated, leaving 43.2 g (51%) of clean 7-methoxycarbonyl-9-(methoxycarbonylmethyl)-9-azabicyclo[3.3.1]nonane-3-one [by thin layer chromatography (5% MeOH in $CH_2Cl_2$) and NMR analysis] as an amber colored, viscous oil.

EXAMPLE 4

Sodium borohydride (2.2 g) was added in equal portions at 10 minute intervals over 1 hour to a cold (0-5 °C), well-stirred solution of 8.8 g (0.032 mol) of 7-methoxycarbonyl- 9-(methoxycarbonylmethyl)-9-azabicyclo[3.3.1]nonane-3-one in 100 ml of methanol. Stirring was continued for an additional 1 hour, after which the methanol was removed at reduced pressure. The residue was dissolved in water (20 ml) and the resulting solution was cooled to 0-5 °C and acidified to pH 1-2 by the addition of 5N hydrochloric acid. This acidic solution was then immediately basified to pH 8-9 with saturated potassium carbonate. Extraction with ethyl acetate (3 x 100 ml) and evaporation of the dried magnesium sulfate extracts afforded 6.9 g (78%) of 7-methoxycarbonyl-9-(methoxycarbonylmethyl)-9-azabicyclo[3.3.1]nonan-3-ol as an amber oil.

EXAMPLE 5

To a well stirred, nitrogen blanketed solution of 8.5 g of 7-methoxycarbonyl-9-(methoxycarbonylmethyl)-9-azabicyclo[3.3.1]nonan-3-ol in 70 ml of methylene chloride and 50 ml of methylal, 2.4 ml (3.5 g, 0.037 mol) of 98% methanesulfonic acid was added dropwise during 2-3 minutes. The resulting mixture was refluxed through 8.5 g of 4A molecular sieves in a Soxhlet apparatus for 20 hours. The resulting solution was basified to about pH 8.0 by the addition of 5N sodium hydroxide and then the bulk of the methylene chloride and methylal was removed at reduced pressure. The residue was diluted with water and extracted with ethyl acetate (2 x 125 ml). Evaporation of the dried sodium sulfate extracts left 8.2 g (84%) of crude 3-methoxymethoxy-7-methoxycarbonyl-9-(methoxycarbonylmethyl)-9-azabicyclo[3.3.1]nonan-3-ol as an amber oil.

EXAMPLE 6

A solution of 26.1 g of crude 7-methoxycarbonyl-9-(methoxycarbonylmethyl)-9-azabicyclo[3.3.1]nonan-3-ol in 250 ml of methylene chloride was treated with one equivalent of methanesulfonic acid (8.42 g). The methylene chloride solution was concentrated to about 35 ml, 9.5 ml of dihydropyran was added together with one drop of methanesulfonic acid, and the mixture stirred for 3 hours at room temperature.

The mixture was then poured into saturated potassium carbonate solution and the product separated by extraction with ethyl acetate.

Evaporation of the dried ethyl acetate extracts gave a syrup consisting mainly of the tetrahydropyranyl ether of 7-methoxycarbonyl-9-(methoxycarbonylmethyl)-9-azabicyclo[3.3.1]nonan-3-ol. It can be purified by column chromatography using silica and elution with hexane-ethyl acetate (20:80).

## EXAMPLE 7

### Method A

Potassium t-butoxide (1.78 g) was added to a well stirred, nitrogen blanketed solution of 4.0 g (12.6 mmol) of 3-methoxymethoxy-7-methoxycarbonyl-9-(methoxycarbonylmethyl)-9-azabicyclo[3.3.1]nonan-3-ol in 60 ml of anhydrous tetrahydrofuran in a reaction flask equipped with a distillation head. The resulting solution was heated to reflux with stirring and refluxed for 2 hours, during which time 25-30 ml of solvent was removed in portions by distillation. During this reflux period, the potassium salt of the Dieckmann product separated. The resulting mixture was cooled to 5-10 °C and, with stirring, diluted with water (20 ml) and ether (20 ml). The aqueous layer was separated and the tetrahydrofuran/ether layer washed with 5 ml of water. The combined aqueous extracts were then acidified by the dropwise addition of 6.5 ml of concentrated hydrochloric acid during 3.5 minutes. This acidic solution was then refluxed for 6 hours. The cooled acidic solution was clarified by an extraction with ethyl acetate (20 ml) then cooled to about 5 °C and basified to pH 8.0-8.5 by the addition of 3.2 g of sodium hydroxide pellets. The resulting solution was saturated by the addition of solid sodium chloride and then continuously extracted with ethyl acetate for 20 hours. The dried (magnesium sulfate) extracts were then evaporated, leaving 1.3 g (57%) of endo-5-hydroxy-8-azatricyclo[5.3.1.$0^{3,8}$]undecan-10-one which solidified and which, by thin layer chromatography (1:1 methanol:methylene chloride) and NMR analysis, was >95% pure.

### Method B

To a well stirred, nitrogen-blanketed solution of potassium t-butoxide, prepared *in situ* from 2.9 g (0.074 g-atom) of clean potassium metal, in a mixture of tert-butyl alcohol (12.2 ml) and tetrahydrofuran (150 ml) in a reaction flask equipped with an addition funnel and distillation head, the tetrahydropyranyl ether of 7-methoxycarbonyl-9-(methoxycarbonylmethyl)-9-azabicyclo[3.3.1]nonan-3-ol (13.0 g, 0.036 mol) was added in one portion. The resulting solution was heated to reflux and refluxed 2 hours, during which a total of 60-75 ml of solvent was removed, in portions, by distillation. The resulting mixture was cooled to 5-10 °C and, with stirring, diluted with water (130 ml) and ether (130 ml). The aqueous layer was separated and the tetrahydrofuran/ ether layer washed with more water (2 x 30 ml). The combined aqueous extracts were then cooled to 0-5 °C and acidified by the dropwise addition of 18.8 ml of concentrated hydrochloric acid during 5-10 minutes. This acidic solution was then refluxed for 6 hours. The cooled acidic solution was clarified by an extraction with ethyl acetate (50 ml) then cooled to 0-5 °C and basified to pH 8.0-8.5 by the addition of sodium hydroxide pellets (about 9.0 g). The resulting solution was saturated by the addition of solid sodium chloride and then continuously extracted with ethyl acetate for 20 hours. The dried (magnesium sulfate) extracts were then evaporated, leaving 5.2 g (79%) of endo-5-hydroxy-8-azatricyclo[5.3.1.$0^{3,8}$]undecan-10-one (of about 95% purity) which solidified.

## EXAMPLE 8

To a well stirred, nitrogen-blanketed solution of 1.4 g (6.66 mmol) of trifluoroacetic anhydride in 25 ml of dry methylene chloride, 1.0 g (6.21 mmol) of indole-3-carboxylic acid was added in equal portions during about 15 minutes. The resulting mixture was stirred 5 minutes after the addi tion was complete, then, with external cooling, 1.2 g (6.62 mmol) of endo-5-hydroxy-8-azatricyclo[5.3.1.$0^{3,8}$]undecan-10-one was added in one portion followed by approximately 10 mg of 4-dimethylaminopyridine. The ice bath was removed after 10-15 minutes and the reaction mixture stirred at room temperature for 20 hours. The mixture was then filtered and the collected solid washed with methylene chloride (5 ml). The solid (2.2 g), which is a mixture of trans-hexahydro-8-(3-indolylcarbonyloxy)-2,6-methano-2H-quinolizin-3(4H)-one trifluoroacetate and unreacted indole-3-carboxylic acid, was dissolved in ethyl acetate (50 ml), washed successively with 10% aqueous sodium carbonate (2 x 10 ml), water (10 ml) and brine (10 ml) and the solution was dried over magnesium sulfate. Evaporation of the solvent left 1.2 g (60%) of trans-hexahydro-8-(3-indolylcarbonyloxy)-2,6-methano-2H-quinolizin-3(4H)-one as a white powder which is pure enough to be used without further purification. Recrystallization of a sample from ethyl acetate-hexane gave analytical material as clusters of fine, colorless needles; m.p. 230-232° C after shrinking at 224° C.

## EXAMPLE 9

To a nitrogen blanketed, filtered solution of 14.5 g (0.045 mol) of trans-hexahydro-8-(3-indolylcarbonyloxy)-2,6-methano-2H-quinolizin-3(4H)-one in 300 ml of reagent grade acetone, there was added 98% methanesulfonic acid (3.1 ml) dropwise, with stirring, during about 5 minutes, while maintaining a reaction temperature of 20-25° C. After a few minutes stirring, trans-hexahydro-8-(3-indolylcarbonyloxy)-2,6-methano-2H-quinolizin-3(4H)-one methanesulfonate began to separate, initially as an oil, solidifying on continued stirring. The mixture was then stirred at 0-5° C for 2 hours, filtered and the collected methanesulfonate washed with acetone (40-50 ml) and dried *in vacuo* at 25° C. The dried trans-hexahydro-8-(3-indolylcarbonyloxy)-2,6-methano-2H-quinolizin-3(4H)-one methanesulfonate weighed 17.1 g (90%). Recrystallization by dissolving in a volume of 15 times its weight of hot 5% aqueous isopropanol, filtering while hot and adding ether to the cloud point when at ambient temperature gave an 85-90% recovery of analytically pure trans-hexahydro-8-(3-indolylcarbonyloxy)-2,6-methano-2H-quinolizin-3(4H)-one methanesulfonate monohydrate.

## EXAMPLE 10

A solution of 6.6 g (31.8 mmol as 100%) of about 85% m-chloroperbenzoic acid in 70 ml of reagent methylene chloride was added dropwise during 30 minutes, under nitrogen, to a cold (0-5° C), well-stirred solution of 3.7 g (29.3 mmol) of methyl 3-cyclopentene-1-carboxylate in 50 ml of methylene chloride. The cooling bath was removed after the addition was complete and the resulting suspension was stirred at room temperature for about 4 hours until the reaction was complete as evidenced by gas chromatographic analysis of an aliquot. The mixture was filtered and the collected m-chlorobenzoic acid washed with 25 ml of methylene chloride. The combined filtrate and wash was washed successively with 10% aqueous sodium bisulfite solution (40 ml), saturated sodium bicarbonate solution (2 x 40 ml) and saturated sodium chloride solution (40 ml) and dried over magnesium sulfate. Concentration left 3.6 g of 1-methoxycarbonyl-3-cyclopentene oxide (86%) as a colorless oil which, by gas chromatographic analysis, was a 3:1 mixture of trans:cis isomers.

## EXAMPLE 11

If the procedure of Example 8 is repeated using, instead of the indole-3-carboxylic acid, a substituted indole-3-carboxylic acid in which the substituent is a 1-methyl, a 7-methyl, a 5-chloro or a 5-bromo, the correspondingly substituted indole ester products are obtained.

## Claims

1. A process for the preparation of a compound of the formula

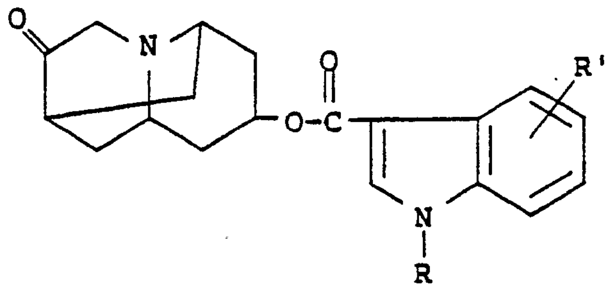

wherein R is hydrogen or $C_{1-4}$ alkyl and R′ is hydrogen, halogen or $C_{1-4}$ alkyl, and the pharmaceutically acceptable acid addition salts thereof, which comprises reacting an alcohol of the formula

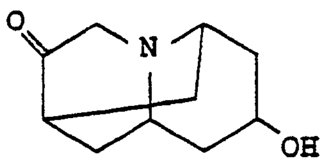

with an anhydride of the formula

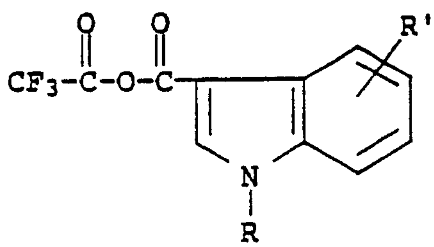

wherein R and R′ are defined as above, in an inert solvent at ambient temperature in the presence of a tertiary amine.

2. A process according to Claim 1 for preparing trans-hexahydro-8-(3-indolylcarbonyloxy)-2,6-methano-2H-quinolizin-3(4H)-one which comprises reacting endo-5-hydroxy-8-azatricyclo[5.3.1.$0^{3,8}$]undecan-10-one with the mixed anhydride of trifluoroacetic acid and indole-3-carboxylic acid.

3. A process according to Claim 1 for preparing trans-hexahydro-8-(3-indolylcarbonyloxy)-2,6-methano-2H-quinolizin-3(4H)-one methanesulfonate which comprises reacting endo-5-hydroxy-8-azatricyclo-[5.3.1.$0^{3,8}$]undecan-10-one with the mixed anhydride of trifluoroacetic acid and indole-3-carboxylic acid to give trans- hexahydro-8-(3-indolylcarbonyloxy)-2,6-methano-2H-quinolizin-3(4H)-one followed by reaction with methanesulfonic acid.

4. A process according to Claim 1 wherein the anhydride is prepared *in situ* from trifluoroacetic anhydride and indole-3-carboxylic acid.

5. A process according to Claim 2 wherein the anhydride is prepared *in situ* from trifluoroacetic anhydride and indole-3-carboxylic acid.

European Patent Office

# EUROPEAN SEARCH REPORT

Application number

EP 89107764.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | EP - A1 - 0 266 730 (MERRELL DOW PHARMACEUTICALS INC.) * Claims 1,9; page 3 * -- | 1 | C 07 D 455/00 C 07 D 471/18// A 61 K 31/445 (C 07 D 471/18, C 07 D 221:00, C 07 D 221:00, C 07 D 221:00) |
| A | HOUBEN-WEYL, Methoden der organischen Chemie, 4. Auflage, Band E5 "Carbonsäuren und Carbonsäure-Derivate" GEORG THIEME VERLAG, Stuttgart, New York pages 691-694 * Pages 691, 694 ---- | 1 | |
| | | | TECHNICAL FIELDS SEARCHED (Int. Cl.4) |
| | | | C 07 D 455/00 C 07 D 471/00 C 07 D 487/00 |

The present search report has been drawn up for all claims

| Place of search | Date of completion of the search | Examiner |
|---|---|---|
| VIENNA | 26-07-1989 | PETROUSEK |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82